# EUROPEAN PATENT APPLICATION

(11) **EP 1 723 920 A2**
(43) Date of publication of application: **22.11.2006**
(21) Application number: 06252607.4
(22) Date of filing: 18.05.2006
(51) Int. Cl.: A61B 17/80, A61B 17/86

(54) **Bone fixation system**

(30) Priority: 20.05.2005 US 134247
(71) Applicant: DePuy Products, Inc., Warsaw, IN 46581 (US)
(72) Inventor: Orbay, Jorge L., Miami, FL 33156 (US); Nunez, Jose Antonio, Miami, FL 33194 (US)
(74) Representative: Belcher, Simon James

(57) **Abstract**

A fracture fixation system (10) for a fracture of a head portion of a long bone has subchondral bone defining a convex articular surface, and particularly the proximal humerus. The system includes both smooth pegs (20) and pegs (22) having a threaded shaft. The shaft (46) of the threaded shaft pegs (22) has both smooth (52) and threaded portions (54). Threaded shaft pegs (22) of different lengths all have the same length threaded portion (54), but different length smooth portions (52). The threaded shaft pegs (22) have a head (44) with an external thread (48). The system further comprises a bone plate (14) which comprises a plurality of threaded holes (40a-f). A drill bit (60) and depth gauge (80) for use with the system are also provided.

## Description

This invention relates broadly to surgical devices. More particularly, this invention relates to bone fasteners, a fracture fixation system including the bone fasteners and an orthopaedic plate.

The proximal humerus comprises the upper portion of the humerus, i.e. upper arm of the human body, and forms a portion of the shoulder joint. Fractures of the proximal humerus typically result from traumatic injuries such as sporting accidents and can be more frequent with age due to bone loss. Fractures of the proximal humerus are treated by exposing the fracture site and reducing the bone fracture and then placing a plate or other means onto the bone to fixate the fracture for healing in the reduced position. Reducing the fracture includes realigning and positioning the fractured portions of the bone to their original position or similar stable position. Fixating the fracture includes positioning a plate over the fractured portions and securing the plate onto the fractured bones and adjacent non-fractured bones with bone screws.

Conventional fixation plate systems have several significant shortcomings when applied to the proximal humerus. Such systems couple the plate to the bone with screws that fail to provide purchase in underlying often osteoporotic bone. As such the screws are prone to loosening from the bone and do not provide the intended support. In addition, particularly in osteoporotic bone, there is a tendency for the screws to push through the bone in which they are set and pierce subchondral bone to enter the articular space between the head of the humerus and the glenoid cavity of the scapula which can cause significant irritation and potentially greater orthopaedic damage. Such damage can interfere with, prolong, or prevent proper healing of the humeral fracture, in addition to causing the patient additional pain and the development of post-traumatic arthritis.

In one aspect, the invention provides a fracture fixation system, comprising:
(a) a bone plate having threaded holes; and
(b) a first set of pegs including pegs of different lengths, each of said pegs having a head and a shaft,
said head having an external thread engaged within one of the threaded holes and a driver engagement means, and
said shaft having a smooth cylindrical portion having a first diameter, and a threaded portion having a major diameter substantially equal to said first diameter, wherein the length of the threaded portion is the same for each of said pegs and the length of the smooth cylindrical portion is different for pegs of different lengths corresponding to the relative length of said pegs.

In another aspect, the invention provides a fracture fixation system, comprising:
(a) a bone plate having threaded holes; and
(b) a first set of pegs, each having a head and a shaft, said head having an external thread engaged within one of the threaded holes and a driver engagement means,
said shaft having a smooth cylindrical portion having a first diameter, and a threaded portion having a major diameter substantially equal to said first diameter and a minor diameter, wherein the ratio of the major to minor diameters is at least 1.5.

In a further aspect, the invention provides a kit for a fracture fixation system, comprising:
(a) a bone plate having threaded holes; and
(b) a first set of pegs including pegs of different lengths, each of said pegs having a head and a shaft,
said head having an external thread for engagement within the threaded holes and a driver engagement means, and
said shaft having a smooth cylindrical portion having a first diameter, and a threaded portion having a major diameter substantially equal to said first diameter, wherein the length of the threaded portion is the same for each of said pegs and the length of the smooth cylindrical portion is different for pegs of different lengths corresponding to the relative length of said pegs.

In yet another aspect, the invention provides a kit for a fracture fixation system, comprising:
(a) a bone plate having threaded holes; and
(b) a first set of pegs each having a head and a shaft, said head having an external thread for engagement within the threaded holes and a driver engagement means, and said shaft having a smooth cylindrical portion having a first diameter, and a threaded portion having a major diameter substantially equal to said first diameter and a minor diameter, wherein the ratio of the major to minor diameters is at least 1.5.

The invention also provides a kit of pegs for bone support, comprising a plurality of pegs of different lengths, each of said pegs having a head and a shaft, said head having an external thread and a driver engagement means, and said shaft having a smooth cylindrical portion having a first diameter, and a threaded portion having a major diameter substantially equal to said first diameter, wherein the length of the threaded portion is the same for each of said pegs and the length of the smooth cylindrical portion is different for pegs of different length corresponding to the relative length of said pegs.

The humeral fracture fixation system of the invention has the advantage that it provides a stable framework for support of a proximal humeral fracture.

The system has the further advantage that fasteners extending through the plate need not break through the articular surface.

The system of screws provided by the invention can facilitate implantation of the humeral fracture fixation system.

Accordingly, the invention provides a humeral fracture fixation system which includes a plate having head and shaft portions, a plurality of pegs for insertion through the head portion and into the humeral head, and a plurality of cortical screws for coupling the shaft portion of the plate to the humeral shaft.

The head portion of the plate is provided with a plurality of threaded holes. A peg is provided for each threaded hole, and extends through the head portion of the plate generally perpendicular to a portion of the articular surface of the humeral head. The pegs are provided in several lengths for humeral heads of different sizes and for the different path lengths within the humeral head defined by the various axes of the threaded holes. The pegs may have a completely smooth shaft, or in accord with the invention have a smooth shaft portion and a threaded shaft portion ('threaded shaft pegs'). In accord with the invention, the threaded shaft pegs regardless of length have a common length threaded shaft portion; however, the length of the smooth shaft portion varies depending on the overall length of the peg. This permits a single step drill bitto be used to drill all the holes for the threaded shaft pegs, regardless of their length, and a single depth gauge to be used therewith as well.

The step drill bit preferably has bone cutting means along first and second portions. The first portion has the same length as the threaded shaft portion of a threaded shaft peg and a first diameter corresponding to the minor diameter of the threaded shaft portion of such peg. The second portion has a second diameter corresponding to the major diameter of the smooth shaft portion of the threaded shaft peg. The drill also has a protruding blunt tip that prevents penetration of the subchondral bone. The depth gauge is also stepped in diameter, having a profile similar to the step drill bit, bit without cutting means. Each of the step drill bit and gauge have indicia thereon to indicate the appropriate length peg to be use in a drilled hole.

Embodiments of the invention will now be described by way of example with reference to the accompanying drawings, in which:
Fig. 1 is an anterior-posterior view of a proximal humeral fixation system according to the invention shown on a proximal humerus;
Fig. 2 is a medial-lateral view of the proximal humeral fixation system according to the invention shown on the proximal humerus;
Fig. 3 is a side elevation view of a set of threaded shaft pegs for use with the proximal humeral fixation system in accord with the invention;
Fig. 4 is a side elevation view of a step drill bit for a threaded shaft peg; and
Fig. 5 is a side elevation view of a depth gauge for determining the appropriate length of a threaded shaft peg.

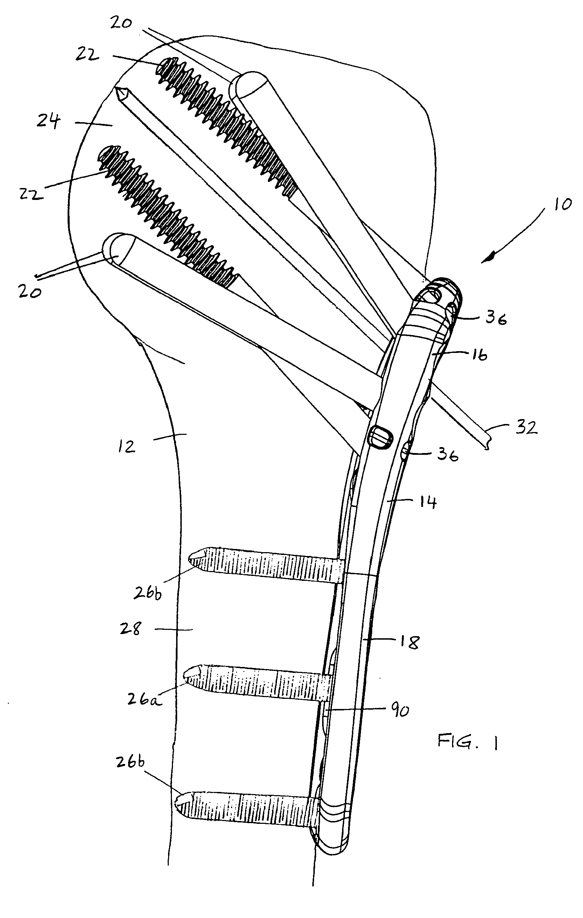

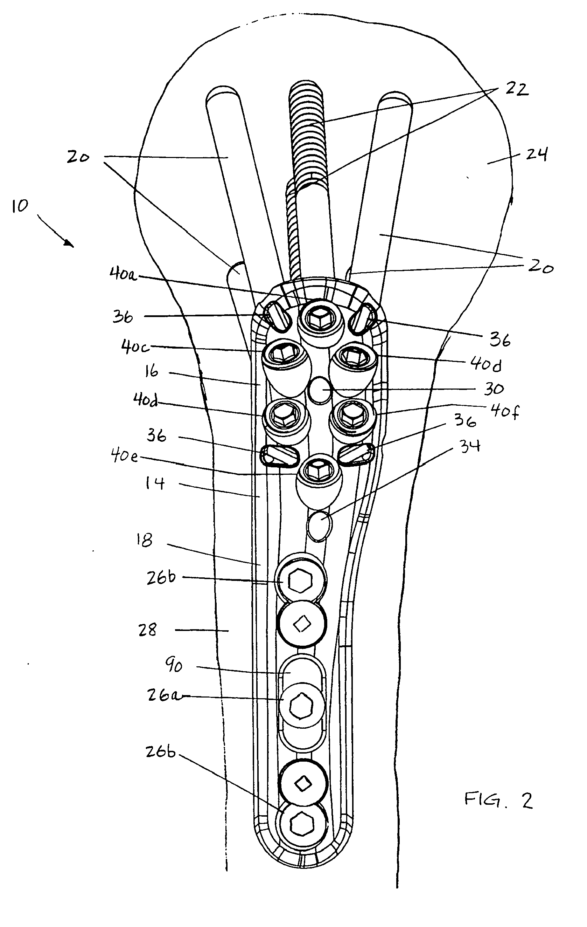

Referring to the drawings, Figs. 1 and 2 show a humeral fracture fixation system 10 on a proximal humerus 12. The system includes a plate 14 having a head portion 16 and shaft portion 18, a plurality of pegs 20, 22 for insertion through the head portion 16 and into the humeral head 24, and a plurality of cortical screws 26a , 26b for coupling the shaft portion 16 of the plate to the humeral shaft 28.

The head portion 16 of plate also includes a central alignment hole 30 for closely receiving a K-wire 32 (Fig. 1), and may also include other alignment holes such as distal alignment hole 34, to hold K-wires at a fixed angle to facilitate alignment of the plate 14 relative to the humerus 12, as described in more detail below. Additionally, suture holes 36 may be provided about the head portion of the plate.

In order to receive the pegs 20, 22, the head portion 16 of the plate is provided with a plurality of threaded holes 40a-f. The threaded holes 40a-f have defined axes. More particularly, proximal and distal threaded holes 40a, 40b have axes which are in the same plane and converge toward a point substantially defined by central alignment hole 30. The axes of holes 40a, 40b are directed substantially perpendicular to the central portion of the articular surface of the humeral head 24. Axes through holes 40c, 40d are directed substantially perpendicular to the upper portion of the articular surface, but diverge to provide support. Axes through holes 40e, 40f are directed substantially perpendicular to the lower portion of the articular surface, and also diverge to provide support; however the divergent angle between the axes through 40e, 40f is smaller than between the axes of 40c, 40d. In addition, the axes through 40e, 40f are also angled relatively further away from the 'centre-line' defined by alignment hole 30, placing the axes therethrough, and thus any pegs therethrough, close to the lowermost part of the articular surface, but orienting such pegs to provide support to prevent the humeral head from going into varus (i.e., in which the lower pegs could protrude through the cortex). Thus, there is an optimal asymmetry to the orientation of the axes (and pegs inserted therethrough).

The pegs 20, 22 may have a completely smooth shaft (smooth shaft pegs 20), or in accord with the invention have a smooth shaft portion and a threaded shaft portion ('threaded shaft pegs' 22). Smooth shaft pegs 20 are used for maximum subchondral support. Threaded shaft pegs 22 may be used in conjunction with the smooth shaft pegs to provide minor lag effect when deemed necessary by the surgeon. The pegs 20, 22 are provided in several lengths for humeral heads of different sizes and for the different path lengths through the bone presented by the different axes of the peg holes. For example, three to eight different lengths may be provided of each of the smooth and threaded shaft pegs 20, 22, and multiple pegs of each length are preferably provided in a kit for assembling the system.

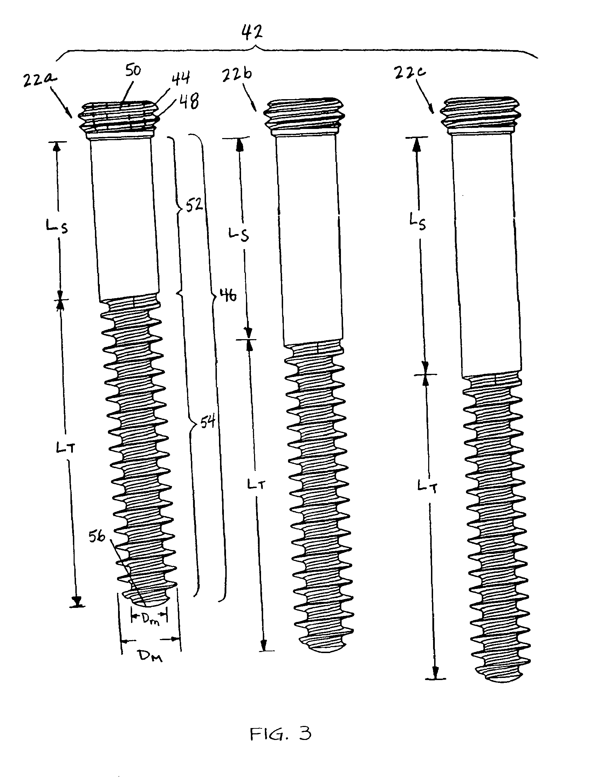

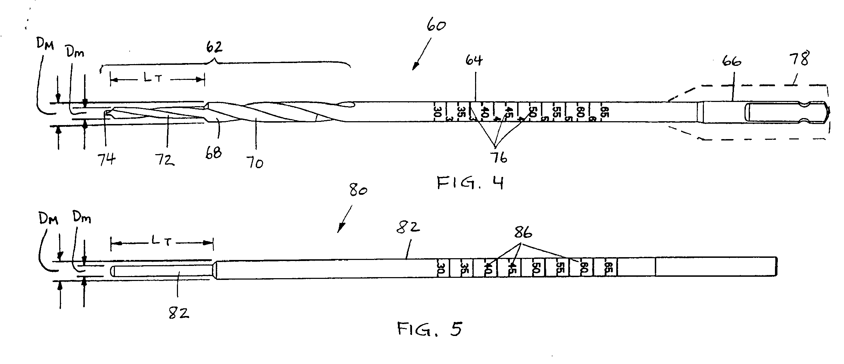

Turning now to Fig. 3, a representative set 42 of three threaded shaft pegs 22a, 22b, 22c, each of different length, are shown. Set 42 is representative, as the set is meant to represent any set of threaded shaft pegs 22 of different lengths, and in which such set may also include multiple numbers of pegs in each of the different lengths. With reference to peg 22a, each threaded shaft peg includes a head 44 and a shaft 46. The head 44 has an external thread 48 and a driver recess 50. The shaft 46 has a smooth preferably cylindrical portion 52 adjacent the head 44, and a threaded portion 54. The end 56 of the shaft 46 is radiused and blunt to provide support to underlying bone and prevent perforation of the bone cortex over time. The outer diameter of the end 56 is substantially equal to the minor diameter of the threaded portion. To further provide support to the bone and to enhance peg fixation, the ratio of the major to minor diameters of the threaded portion 54 is relatively large; preferably at least 1.5, more preferably 1.65, and most preferably approximately 1.8, but can be greater. By way of example, the major diameter (D_{M}) of peg 22a is 0.157 inch and the minor diameter (Dₘ) is 0.087 inch, providing a ratio of 1.8. This results in a peg shaft that has increased bone purchase and resistance to pull-out, similar to a cancellous screw, in distinction from the cortical-type screws commonly used in humeral systems which have shallow threads along the shaft. Moreover, the pitch of the threads on the shaft are substantially similar to the pitch of the external threads 48 on the head 44, thereby minimizing undesirable compression. Each peg 22a, 22b, 22c has the same length L_{T}, regardless of the overall length of the peg. It is the length Lₛ of the smooth portion 52 of the peg that varies relative to the other pegs in the set 42 depending on the overall length of the peg. This feature permits a single step drill bit 60 (Fig. 4) and a single depth gauge 80 (Fig. 5) to be used to drill all the holes for the threaded shaft pegs, regardless of the length of the pegs.

Referring to Fig. 4, the step drill bit 60 includes a stepped working end 62, a midshaft 64, and a driver engageable end 66. The working end 62 includes cutting flutes 68 or other structure adapted to remove bone, preferably upon rotation. The working end 62 includes a first portion 70 adjacent the midshaft 64 with the same diameter as both the smooth portion 52 and the major diameter D_{M} of the threaded shaft portion 54 of the threaded shaft peg 22 (Fig. 3). The working end 62 also includes a relatively distal second portion 72 having the same length L_{T} as the threaded shaft portion 54 of a threaded shaft peg 22, and a second smaller diameter corresponding to the minor diameter Dₘ of the threaded shaft portion 54 of such peg 22. The working end 62 is also provided with a protruding blunt tip 74, approximately 3 mm in length, which does not inhibit the drill moving through soft bone in the humeral head but which will contact the far cortex and prevent the cutting flutes from contacting and penetrating the subchondral cortex bone. The diameter of the blunt tip is smaller than minor diameter Dₘ. The midshaft 64 includes a scale 76 which, upon drilling a hole to desired depth, indicates the length of the threaded shaft peg which should be used in the drilled hole. The step drill bit 60 may be provided in an alternate version wherein the driver engageable end 66 is replaced with a manual grip 78 (shown in broken lines) to facilitate manual drilling.

The depth gauge 80 has a similar profile to the step drill bit 60, but no bone cutting means. That is, the gauge 80 includes a shaft 82 with a stepped end (reduced diameter portion) 84. The stepped end 84 has a length corresponding to L_{T}. The shaft has diameter corresponding to D_{M}, and the stepped end 84 has a diameter corresponding to Dₘ. The shaft includes a scale 86 that indicates the length of the threaded shaft peg which should be used within a drilled hole into which the gauge is inserted.

A delto-pectoral approach is developed to expose and debride the fracture. Traction and direct manipulation are then used to reduce the fracture. The anatomical relationship between the articular surface and the humeral shaft are reestablished by restoring both angular alignment and rotation. Tuberosities are examined for assurance that they can be reduced to their proper position.

The position of the plate is then located, preferably immediately lateral to the intertubercle groove and approximately 2.5 cm below the insertion of the supraspinatus.

Referring back to Figs. 1 and 2, the plate is secured to the distal fragment using a cortical screw 26a inserted through the non-locking oblong screw hole 90 to or a plate-holding clamp. The reduction is then locked using a K-wire 32 (e.g., 2.0 mm) inserted through the central fixed angle k-wire hole 30 on the head portion 16 of the plate 14 and into the proximal fragment(s) of the humeral head 24. The K-wire 32 fixes the fracture and anticipates the final position of the pegs 20, 22. The reduced fracture, plate location and K-wire are then evaluated using fluoroscopy (preferably both AP and axillary views) and readjusted as necessary.

Using a short first drill bit under power, the lateral cortex is penetrated to start the peg holes 40a-f within the bone. Drill guides are preferably aligned relative to the peg hole axes to facilitate drilling the remainder of the hole at the proper axial orientation. For smooth shaft pegs 20, a different non-stepped drill bit (not shown) is then used to drill the rest of the holes to the appropriate the depth. Such drill bit has all of the features described with respect to step drill bit 60, but the working end has a constant diameter D_{M}, with the optional provision of the protruding blunt tip. For threaded shaft pegs 22, the step drill bit 60 is used to the drill the holes to appropriate depth. In accord with the invention, the drilling of the holes through the humeral head after penetration of the cortex is performed entirely by hand, by manual manipulation of the bit. The cancellous bone within the central region of the humeral head is relatively soft and easy to drill through under manual manipulation of the drill bit. While fluoroscopy is preferably used to prevent penetration of the subchondral bone, manual drilling provides sufficient tactile feedback of when the drill bit reaches the far cortex that fluoroscopy is not essential to determine when the hole is of proper depth. Particularly, the protruding blunt tip 74 (Fig. 4) functions as a stop against the hard far cortex at the appropriate hole depth.

The depth of drilled stepped holes for the threaded shaft pegs 22 can be determined from the scale on the step drill bit 60. Alternatively, the step drill bit 60 can be removed from the hole, and the depth gauge 80 can be inserted into the hole to determine the depth of the drilled hole. The depth of drilled holes for the smooth pegs can be determined with a constant diameter depth gauge (not shown) or depth gauge 80. The appropriate length and type of pegs (smooth shaft and/or threaded shaft) are selected and inserted using a driver and secured to the fixation plate. The distal end of the pegs should be 3 to 6 mm below the subchondral bone.

After peg placement, radiographic confirmation of correct fracture reduction and peg placement is preferably obtained. Then using a drill bit, holes are drilled for the remaining cortical screws 26 that will be used to fix the distal shaft portion 18 of the plate 14 to the diaphysis (shaft) 18 of the humerus. For the humeral shaft, either multidirectional screws 26a or fixed angle screws 26b can be used.

Then, if necessary, tuberosities are reduced and fixed to the plate at the suture holes 36 using sutures or wires. Finally, the surgical site is closed using appropriate surgical technique.

While the described embodiment is for a humeral fracture fixation system, it is appreciated that the system is well adapted to bone fractures of any articular surface having a convex shape. Thus, the system of the invention could similarly be used to treat, e.g., a fracture of the femoral head. Moreover, while the system has been described for use with respect to fractures, it is appreciated that it may also be used in the treatment of osteotomies and non-unions of the proximal humerus and other bones having an articular surface with a convex shape. While the system has been described with respect to using both smooth and threaded shaft pegs together, it is appreciated that only smooth pegs, or only threaded shaft pegs may be used to fix the plate to bone.

## Claims

**1.** A fracture fixation system, comprising:
(a) a bone plate having threaded holes; and
(b) a first set of pegs including pegs of different lengths, each of said pegs having a head and a shaft,
said head having an external thread engaged within one of the threaded holes and a driver engagement means, and
said shaft having a smooth cylindrical portion having a first diameter, and a threaded portion having a major diameter substantially equal to said first diameter, wherein the length of the threaded portion is the same for each of said pegs and the length of the smooth cylindrical portion is different for pegs of different lengths corresponding to the relative length of said pegs.

**2.** A system according to claim 1, in which an end of each of said pegs has an outer diameter which is radiused and blunt.

**3.** A system according to claim 1, in which said threaded portion of said shaft of each said peg of said first set defines a minor diameter, and a ratio of major to minor diameters is at least 1.5, preferably at least 1.65, more preferably at least 1.8.

**4.** A system according to claim 1, in which said threaded shaft portion includes threads which are substantially similar in pitch said external threads on said head of said peg.

**5.** A system according to claim 1, which includes a second set of pegs, each of said pegs having a non-threaded shaft and a head with an external thread and a driver engagement means.

**6.** A system according to claim 1, in which said threaded holes have axes which are oriented in relatively oblique axes.

**7.** A system according to claim 6, in which two of said axes are directed toward a common point.

**9.** A system according to claim 7, in which said two axes are in a common plane.

**10.** A system according to claim 7, in which at least two of others of said axes are divergent.

**11.** A fracture fixation system, comprising:
(a) a bone plate having threaded holes; and
(b) a first set of pegs, each having a head and a shaft, said head having an external thread engaged within one of the threaded holes and a driver engagement means,
said shaft having a smooth cylindrical portion having a first diameter, and a threaded portion having a major diameter substantially equal to said first diameter and a minor diameter, wherein the ratio of the major to minor diameters is at least 1.5.

**12.** A kit for a fracture fixation system, comprising:
(a) a bone plate having threaded holes; and
(b) a first set of pegs including pegs of different lengths, each of said pegs having a head and a shaft,
said head having an external thread for engagement within the threaded holes and a driver engagement means, and
said shaft having a smooth cylindrical portion having a first diameter, and a threaded portion having a major diameter substantially equal to said first diameter, wherein the length of the threaded portion is the same for each of said pegs and the length of the smooth cylindrical portion is different for pegs of different lengths corresponding to the relative length of said pegs.

**13.** A kit for a fracture fixation system, comprising:
(a) a bone plate having threaded holes; and
(b) a first set of pegs each having a head and a shaft, said head having an external thread for engagement within the threaded holes and a driver engagement means, and said shaft having a smooth cylindrical portion having a first diameter, and a threaded portion having a major diameter substantially equal to said first diameter and a minor diameter, wherein the ratio of the major to minor diameters is at least 1.5.

**14.** A kit of pegs for bone support, comprising a plurality of pegs of different lengths, each of said pegs having a head and a shaft, said head having an external thread and a driver engagement means, and said shaft having a smooth cylindrical portion having a first diameter, and a threaded portion having a major diameter substantially equal to said first diameter, wherein the length of the threaded portion is the same for each of said pegs and the length of the smooth cylindrical portion is different for pegs of different length corresponding to the relative length of said pegs.

**15.** A peg for bone support, comprising a head portion having an external thread and a driver recess, and a shaft having a smooth portion with a first diameter, and a threaded portion defining a major diameter and a minor diameter, wherein said major diameter is substantially equal to said first diameter and a ratio of the major to minor diameters is at least 1.5.
